# EUROPÄISCHE PATENTSCHRIFT

(11) **EP 1 109 802 B1**
(45) Veröffentlichungstag und Bekanntmachung des Hinweises auf die Patenterteilung: **10.04.2002**
(21) Anmeldenummer: 00947907.2
(22) Anmeldetag: 30.06.2000
(51) Int. Cl.: C07D 311/96, C07D 493/10, C07D 495/10, G02B 5/23, C08K 5/15

(54) **SPIROFLUORENOPYRANE**
SPIROFLUORENOPYRANS
SPIROFLUORENEPYRANNES

(30) Priorität: 02.07.1999 DE 19930290
(43) Veröffentlichungstag der Anmeldung: 27.06.2001
(73) Patentinhaber: Optische Werke G. Rodenstock, 80469 München (DE)
(72) Erfinder: MANN, Claudia, D-80634 München (DE); MELZIG, Manfred, D-82234 Wessling (DE); WEIGAND, Udo, D-80638 München (DE)
(74) Vertreter: Müller-Boré & Partner Patentanwälte
(86) Internationale Anmeldenummer: EP0006156
(87) Internationale Veröffentlichungsnummer: WO0102384

(56) Entgegenhaltungen:
- WO-A-96/14596
- US-A- 5 869 658

## Beschreibung

Die vorliegende Erfindung betrifft spezifische photochrome 3H-Naphtho[2,1-b]-pyranderivate sowie deren Verwendung in Kunststoffen aller Art, insbesondere für ophthalmische Zwecke. Insbesondere betrifft die vorliegende Erfindung von Naphthopyranen abgeleitete Spiroverbindungen mit Fluorenstruktur, sogenannte Spirofluorenopyrane, bei denen am zentralen Kohlenstoffatom der Fluorenstruktur ein annelliertes Ringsystem über eine Spiroverknüpfung gebunden ist.

Es sind verschiedene Farbstoffklassen bekannt, die bei Bestrahlung mit Licht bestimmter Wellenlängen, insbesondere Sonnenstrahlen, reversibel ihre Farbe wechseln. Dies rührt daher, daß diese Farbstoffmoleküle durch Energiezufuhr in Form von Licht in einen angeregten farbigen Zustand übergehen, den sie bei Unterbrechung der Energiezufuhr wieder verlassen, wodurch sie in ihren farblosen oder zumindest kaum gefärbten Normalzustand zurückkehren. Zu diesen photochromen Farbstoffen gehören beispielsweise die Naphthopyrane, die im Stand der Technik mit verschiedenen Substituenten bereits beschrieben wurden.

Pyrane, speziell Naphthopyrane und von diesen abgeleitete größere Ringsysteme, sind photochrome Verbindungen, die bis heute Gegenstand intensiver Untersuchungen sind. Obwohl bereits im Jahr 1966 erstmals zum Patent angemeldet (US 3,567,605), konnten erst in den 90er Jahren Verbindungen entwickelt werden, die für den Einsatz in Brillengläsern geeignet erschienen.

Von 2-Naphtholen abgeleitete 3H-Naphthopyrane und ihre davon durch Annellierung abgeleiteten höheren analogen Derivate sind eine Gruppe von photochromen Farbstoffen, deren längstwelliges Absorptionsmaximum der angeregten Form vorwiegend im Spektralbereich von 420 nm bis 500 nm liegt und somit einen gelben, orangen oder roten Farbeindruck vermitteln.

Die Geschwindigkeit der Aufhellung wird dabei durch sterische Hinderung oder durch elektronische Effekte beeinflußt. Hierbei sind vor allem die Substituenten an den Arylresten an dem Kohlenstoffatom von Bedeutung, das dem Pyran-Sauerstoffatom benachbart ist. Der sterische Einfluß durch Substituenten in ortho-Stellung solcher Arylreste ist beispielsweise in US-A 5,066,818 beschrieben. Der elektronische Einfluß von Substituenten an diesen Arylringen oder am Naphthalinrest selbst wirkt sich sowohl auf das Absorptionsmaximum als auch auf die Aufhellgeschwindigkeit aus. Dies ist beispielsweise in US-A 5,520,853 und US-A 5,623,005 sowie WO 99/31082 beschrieben.

Die im Stand der Technik bekannten, von 2-Naphtholen abgeleiteten 3H-Naphthopyrane und ihre davon durch Annellierung abgeleiteten höheren analogen Derivate sind jedoch mit Nachteilen verbunden, die bei deren Verwendung in Sonnenschutzgläsern den Tragekomfort des Brillenträgers wesentlich beeinträchtigen. Die im Stand der Technik bekannten Farbstoffe weisen dabei zum einen häufig eine nicht ausreichend langwellige Absorption im angeregten wie im nicht angeregten Zustand auf. Dies führt auch bei Kombinationen mit weiteren photochromen Farbstoffen zu Problemen. Zum anderen liegt häufig auch eine zu hohe Temperaturempfindlichkeit bezüglich der Eindunkelung vor, wobei gleichzeitig eine zu langsame Aufhellung eintritt. Darüberhinaus besitzen die beschriebenen Farbstoffe eine ungenügende Lebensdauer. Infolgedessen zeigen derartige Sonnenschutzgläser eine unzureichende Haltbarkeit. Letzteres macht sich in schnell nachlassender Leistung und/oder starker Vergilbung bemerkbar.

Somit liegt der vorliegenden Erfindung die Aufgabe zugrunde, neue photochrome 3H-Naphthopyrane bereitzustellen, die verbesserte Eigenschaften gegenüber den im Stand der Technik beschriebenen Verbindungen besitzen sollen. Die photochromen Verbindungen sollen sich gegenüber vergleichbaren Verbindungen aus dem Stand der Technik insbesondere durch eine schnellere Kinetik in der angeregten Form, d.h. schnellere Aufhellungsgeschwindigkeit, sowie durch ein besseres Verhalten im Lebensdauertest auszeichnen.

Diese Aufgabe wird durch die in den Ansprüchen gekennzeichneten Gegenstände gelöst. Insbesondere werden photochrome 3H-Naphtho-[2,1-b]pyrane mit der allgemeinen Formel (I) bereitgestellt: 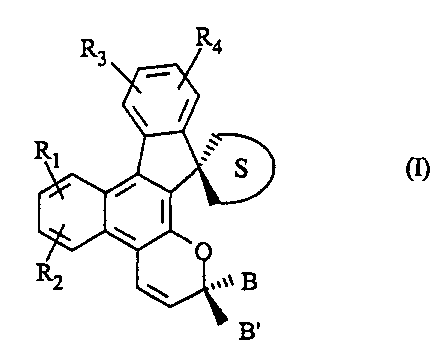 worin
die Reste R₁, R₂, R₃ und R₄ jeweils unabhängig voneinander einen Substituenten darstellen, ausgewählt aus der Gruppe A, bestehend aus einem Wasserstoffatom, einem (C₁-C₆)-Alkylrest, einem (C₁-C₆)-Alkoxyrest, einem (C₃-C₇)-Cycloalkylrest, der ein oder mehrere Heteroatome aufweisen kann, einem Phenylrest, einer Hydroxygruppe, Brom, Chlor und Fluor;
die Struktureinheit S unter Einbeziehen des zentralen Spirokohlenstoffatoms ein gesättigtes und/oder ungesättigtes Ringglied mit 5 bis 8 Kohlenstoffatomen darstellt, von denen maximal eines durch ein Heteroatom, ausgewählt aus der Gruppe, bestehend aus O, S und NR₅, ersetzt sein kann, wobei der Rest R₅ einen geradkettigen oder verzweigtkettigen (C₁-C₆)-Alkylrest, einen substituierten oder unsubstituierten (C₅-C₇)-Cycloalkylrest, einen substituierten oder unsubstituierten Phenylrest oder einen substituierten oder unsubstituierten Benzylrest darstellt, wobei an das Ringglied mindestens ein aromatisches oder heteroaromatisches Ringsystem annelliert ist, wobei das Ringsystem ausgewählt ist aus der Gruppe E, bestehend aus Benzol, Naphthalin, Phenanthren, Pyridin, Chinolin, Furan, Thiophen, Pyrrol, Benzofuran, Benzothiophen, Indol und Carbazol, und das Ringsystem einen oder mehrere Substituenten aus der Gruppe A aufweisen kann;
B und B' unabhängig voneinander aus einer der folgenden Gruppen a), b), c) oder d) ausgewählt sind, wobei sie
a) mono-, di- und trisubstituierte Arylreste sind, wobei der Arylrest Phenyl oder Naphthyl ist;
b) unsubstituierte, mono- und disubstituierte Heteroarylreste sind, wobei der Heteroarylrest Pyridyl, Furanyl, Benzofuran-2-yl, Benzofuran-3-yl, Thienyl, Benzothien-2-yl oder Benzothien-3-yl ist;
   wobei die Substituenten der Aryl- oder Heteroarylreste in a) und b) solche sind, ausgewählt aus einer Gruppe, bestehend aus Hydroxy, Amino, Mono-(C₁-C₆)-alkylamino, Di-(C₁-C₆)-alkylamino, am Aromaten un-, mono- oder disubstituiertem Mono- und Diphenylamino, Piperidinyl, Morpholinyl, Carbazolyl, un-, mono- und disubstituiertem Pyrryl, (C₁-C₆)-Alkyl, (C₁-C₆)-Alkoxy, Brom, Chlor und Fluor, wobei die vorgenannten aromatischen und heteroaromatischen Ringsysteme mit (C₁-C₆)-Alkyl, (C₁-C₆)-Alkoxy, Brom, Chlor und Fluor substituiert sein können;
c) Struktureinheiten mit den folgenden Formeln (V) und (W) sind: 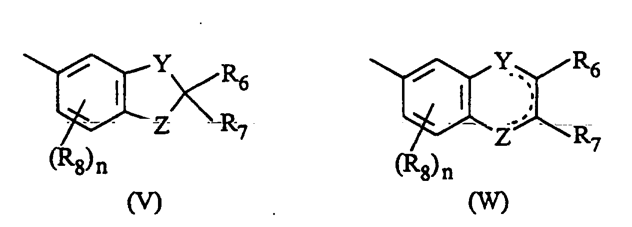 worin
   Y und Z unabhängig voneinander O, S, CH, CH₂ oder NR₉ sind, wobei der Rest R₉ ausgewählt ist aus der Gruppe D, bestehend aus (C₁-C₆)-Alkyl, (C₁-C₆)-Acyl und Wasserstoff, die Reste R₆ und R₇ unabhängig voneinander Wasserstoff und/oder einen (C₁-C₆)-Alkylrest darstellen und der Rest R₈ ein Substituent aus der Gruppe A ist, wobei n 1, 2, oder 3 ist, mit der Maßgabe, daß, wenn Y in der Formel (V) NR₉ ist, Z Kohlenstoff ist,
   oder
d) B und B' miteinander einen un-, mono- oder disubstituierten Fluoren-9-ylidenrest oder einen gesättigten Kohlenwasserstoffrest bilden, der C₃-C₁₂ spiro-monozyklisch, C₇-C₁₂ spiro-bizyklisch und/oder C₇-C₁₂ spiro-trizyklisch ist, wobei die Fluorensubstituenten aus der Gruppe A ausgewählt sind.

In einer bevorzugten Ausführungsform ist die Struktureinheit S in der vorstehenden Formel (I) unter Einbeziehen des zentralen Spirokohlenstoffatoms eine substituierte oder unsubstituierte Fluoren-, Xanthen-, Thioxanthen- oder Dihydroanthracen-Einheit.

Bevorzugte photochrome 3H-Naphtho[2,1-b]pyrane weisen die nachfolgende allgemeine Formeln (II) auf: 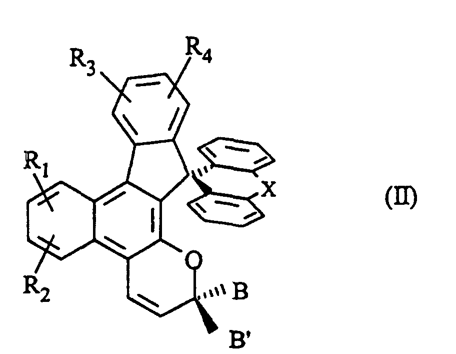 worin R₁, R₂, R₃ und R₄ wie vorgenannt definiert sind und X für -CH₂-, -O-, -Soder eine C-C-Einfachbindung zwischen den Phenylringen steht.

In einer besonders bevorzugten Ausführungsform sind in der vorstehend aufgeführten Formel (II) B und B' unabhängig voneinander mono-, di- und trisubstituierte Arylreste, wobei der Arylrest jeweils ein Phenylrest oder ein Naphthylrest ist.

Besonders bevorzugte Verbindungen gemäß der vorliegenden Erfindung sind:
Spiro-9-fluoren-13'-[6-methoxy-2-(4-methoxyphenyl)-2-phenyl-indeno[2,1-f]naphtho[2,1-b]pyran],
Spiro-9-xanthen-13'-[6-methoxy-2-(4-methoxyphenyl)-2-phenyl-indeno[2,1-f]naphtho[2,1-b]pyran],
Spiro-9-thioxanthen-13'-[6-methoxy-2-(4-methoxyphenyl)-2-phenyl-indeno[2,1-f]-naphtho[2,1-b]pyran],
Spiro-9-(9,10-dihydroanthracen)-13'-[6-methoxy-2-(4-methoxyphenyl)-2-phenylindeno[2,1-f]naphtho[2,1-b]pyran] und
Spiro-9-fluoren-13'-[6,7-dimethoxy-2-(4-methoxyphenyl)-2-phenyl-indeno[2,1-f]-naphtho[2,1-b]pyran].

Gemäß der vorliegenden Erfindung werden durch spirocyclische Ringbildung an der Fluorenostruktur des Naphthopyrangerüsts Verbindungen bereitgestellt, die sich gegenüber den im Stand der Technik bekannten Verbindungen sowohl durch ein besseres Alterungsverhalten als auch durch eine schnellere Aufhellung aus dem eingedunkelten Zustand auszeichnen.

EP-A-0 987 260 beschreibt zwar den positiven Einfluß, den eine Verbrückung bei Indeno-annellierten Naphthopyranderivaten, die sich vom 1-Naphtholsystem ableiten, auf die Lebensdauer hat. Wendet man diese Verbrückung auf die von 2-Naphtholen abgeleiteten annellierten Ringsysteme, wie in US 5,869,658 beschrieben, an, so ist auch hier eine deutliche Verbesserung der Lebensdauer zu erwarten. Abweichend von den Verbindungen der EP-A 0 987 260 ist jedoch kein Einfluß auf die Aufhellgeschwindigkeit der erfindungsgemäßen Verbindungen zu erwarten, da sich hier kein sterischer Einfluß bei der Ringschließung des Pyranrings ergibt. Obwohl die erfindungsgemäßen Verbindungen im Vergleich zu den in US 5,869,658 beschriebenen Verbindungen sterisch deutlich anspruchsvoller substituiert sind, ist jedoch überraschenderweise ihre Aufhellgeschwindigkeit gegenüber den in US 5,869,658 beschriebenen Verbindungen viel höher. Besonders bemerkenswert ist dabei, daß, wenn die Struktureinheit S in der allgemeinen Formel (I) eine Fluoren-, Xanthen-, Thioxanthen- oder Dihydroanthracen-Einheit ist, durch eine geringfügige gegenseitige Abspreizung der beiden Phenylringe der vorgenannten Struktureinheiten durch den Substituenten X in der allgemeinen Formel (II) die Aufhellgeschwindigkeit annähernd um den Faktor 3 verändert werden kann. Die erfindungsgemäße Verknüpfung zu Spirosystemen führt damit nicht nur zu einer Verbesserung der Lebensdauer, sondern eröffnet gleichzeitig auch eine einfache Möglichkeit die Aufhellgeschwindigkeit zu beeinflussen, ohne dabei gleichzeitig durch elektronische Effekte das Absorptionsmaximum zu ändern.

Die erfindungsgemäßen Verbindungen können in Kunststoffmaterialien bzw. Kunststoffgegenständen jeglicher Art und Form für eine Vielzahl von Einsatzzwecken, für die photochromes Verhalten von Bedeutung ist, verwendet werden. Dabei können ein Farbstoff gemäß der vorliegenden Erfindung oder ein Gemisch solcher Farbstoffe eingesetzt werden. Beispielsweise können die photochromen Naphthopyran-Farbstoffe gemäß der vorliegenden Erfindung in Linsen, insbesondere ophthalmischen Linsen, Gläsern für Brillen aller Art, wie beispielsweise Skibrillen, Sonnenbrillen, Motorradbrillen, Visieren von Schutzhelmen, und dergleichen eingesetzt werden. Ferner können die erfindungsgemäßen Naphthopyrane beispielsweise auch als Sonnenschutz in Fahrzeugen und Wohnräumen in Form von Fenstern, Schutzblenden, Abdeckungen, Dächern oder dergleichen verwendet werden.

Zur Herstellung von solchen photochromen Gegenständen können die erfindungsgemäßen photochromen Naphthopyran-Farbstoffe durch verschiedene, im Stand der Technik beschriebene Verfahren, wie bereits in WO 99/15518 angegeben, auf ein Polymermaterial, wie ein organisches Kunststoffmaterial, aufgebracht oder darin eingebettet werden.

Es werden dabei sogenannte Massefärbungs- und Oberflächenfärbungsverfahren unterschieden. Ein Massefärbungsverfahren umfaßt beispielsweise das Auflösen oder Dispergieren der photochromen Verbindung oder Verbindungen gemäß der vorliegenden Erfindung in einem Kunststoffmaterial, z.B. durch die Zugabe der photochromen Verbindung(en) zu einem monomeren Material, bevor die Polymerisation erfolgt. Eine weitere Möglichkeit zur Herstellung eines photochromen Gegenstands ist die Durchdringung des oder der Kunststoffmaterialien mit der (den) photochromen Verbindung(en) durch Eintauchen des Kunststoffmaterials in eine heiße Lösung des oder der photochromen Farbstoffe gemäß der vorliegenden Erfindung oder beispielsweise auch ein Thermotransferverfahren. Die photochrome(n) Verbindung(en) kann bzw. können beispielsweise auch in Form einer separaten Schicht zwischen aneinandergrenzenden Schichten des Kunststoffmaterials, z.B. als Teil eines polymeren Films, vorgesehen werden. Ferner ist auch ein Aufbringen der photochromen Verbindung(en) als Teil einer auf der Oberfläche des Kunststoffmaterials befindlichen Beschichtung möglich. Der Ausdruck "Durchdringung" soll dabei die Migration der photochromen Verbindung(en) in das Kunststoffmaterial, z.B. durch den lösungsmittelunterstützten Transfer der photochromen Verbindung(en) in eine Polymermatrix, Dampfphasentransfer oder andere derartige Oberflächendiffusionsvorgänge, bedeuten. Vorteilhafterweise können solche photochromen Gegenstände, wie z.B. Brillengläser, nicht nur mittels der üblichen Massefärbung, sondern in gleicher Weise auch mittels Oberflächenfärbung hergestellt werden, wobei bei der letzteren Variante eine überraschend geringere Migrationsneigung erzielt werden kann. Dies ist vor allem bei nachfolgenden Veredelungsschritten von Vorteil, da - z.B. bei einer Antireflexbeschichtung durch die geringere Rückdiffusion im Vakuum - Schichtablösungen und ähnliche Defekte drastisch verringert werden.

Insgesamt können auf Basis der erfindungsgemäßen photochromen 3H-Naphtho-[2,1-b]pyrane beliebig kompatible (in chemischer Hinsicht und farblicher Art und Weise verträgliche) Färbungen, d.h. Farbstoffe, auf das Kunststoffmaterial aufgebracht oder in es eingebettet werden, um sowohl ästhetischen Gesichtspunkten als auch medizinischen oder modischen Aspekten zu genügen. Der oder die spezifisch ausgewählte(n) Farbstoff(e) kann bzw. können demzufolge, abhängig von den beabsichtigten Wirkungen sowie Anforderungen, variieren.

Der Syntheseweg der erfindungsgemäßen Spiroverbindungen ist im Vergleich zu den in US-A 5,869,658 beschriebenen Verfahren erheblich modifiziert. Während zur Synthese gemäß US-A 5,869,658 (vgl. auch die in Tabelle 1 angeführte Vergleichsverbindung) das Fluorenon-Zwischenprodukt durch Oxidation des entsprechenden Fluorens gewonnen werden muß, wird es im Rahmen der vorliegenden Erfindung direkt bei der Cyclisierung hergestellt. Ausgehend von Benzylcyanid-Derivaten werden die entsprechenden 2-Acetyl-Verbindungen gemäß Schritt i) durch Esterkondensation mit Essigester hergestellt. Nach Verseifung und Decarboxylierung werden die entsprechenden Phenylaceton-Derivate (Schritt ii) hergestellt. Durch doppelte Esterkondensation mit Phthalsäuredimethylester (Schritt iii)) entstehen 2-Phenylacetylindandion-Derivate, die wiederum Ausgangsstoffe für die intramolekulare Cyclisierung zu den gewünschten Hydroxyfluorenon-Derivaten gemäß Schritt iv) bilden. Diese werden schließlich mit 2-Propin-1-ol-Derivaten gemäß Schritt v) zu Indeno-annellierten Naphthopyran-Derivaten umgesetzt. Dieser Schritt sowie die beiden anschließenden Schritte erfolgen analog zu den Umsetzungen mit isomeren Hydroxyfluorenon-Derivaten, wie in EP-A-0 987 260 beschrieben. In Schritt vi) erfolgt die Umsetzung mit geeigneten Grignard-Reagenzien, die sich in Schritt vii) zu den erfindungsgemäßen Spiroverbindungen cyclisieren lassen.

Nachfolgend wird die Herstellung beispielhaft ausgewählter, erfindungsgemäßer 3H-Naphtho[2,1-b]pyrane detailliert erläutert, wobei diese Beispiele selbstverständlich den Schutzbereich der vorliegenden Erfindung nicht beschränken, sondern nur zur Veranschaulichung dienen sollen.

### Beispiele

### BEISPIEL 1

i) Eine Mischung aus 3-Methoxybenzylcyanid (100 g), Essigsäureethylester (90 g) und Kaliumethylat (78 g) in 1000 ml absolutem Toluol wurde 4 h unter Rühren und Rückfluß zum Sieden erhitzt. Anschließend wurden 600 ml Wasser zugegeben und die organische Phase im Scheidetrichter abgetrennt. Diese wurde nochmals mit 600 ml Wasser extrahiert. Die vereinigten wäßrigen Phasen wurden zweimal mit Ether extrahiert und anschließend wurde die wäßrige Phase mit konz. Salzsäure angesäuert. Es wurde zweimal mit je 400 ml Ether extrahiert und die vereinigte organische Phase mit Wasser und verdünnter Natriumhydrogencarbonat-Lösung gewaschen. Nach Trocknen über Natriumsulfat und Abdestillieren des Lösungsmittels wurde ein viskoses Öl erhalten (103 g), das bei Raumtemperatur erstarrte und mittels NMR-Spektrum als 2-(3-Methoxyphenyl)-3-oxo-butansäurenitril identifiziert wurde.
ii) Eine Mischung aus 175 ml konz. Schwefelsäure und 50 ml Wasser wurde im Eisbad unter Rühren abgekühlt. Nach Zugabe des in Schritt i) erhaltenen Reaktionsproduktes (100 g) wurde kurz bis zur Lösung erhitzt. Anschließend wurde wieder im Eisbad abgekühlt und 900 ml Wasser zugegeben. Das Reaktionsgemisch wurde 6 h unter Rühren und Rückfluß zum Sieden erhitzt. Nach dem Abkühlen wurde zweimal mit 400 ml Ether extrahiert und die vereinigte organische Phase wurde zweimal mit Wasser und verdünnter Natriumhydrogencarbonat-Lösung gewaschen. Nach Trocknen über Natriumsulfat und Abdestillieren des Lösungsmittels wurde das Rohprodukt einer Säulenchromatographie mit Aluminiumoxid (Wassergehalt 3%) als stationärer und Dichlormethan als mobiler Phase unterworfen. Das Produkt war ein gelbliches Öl (45 g), das mittels NMR-Spektroskopie als 3-Methoxyphenylaceton identifiziert wurde.
iii) Ein Gemisch aus dem in Schritt ii) erhaltenen Reaktionsprodukt (33 g), Phthalsäuredimethylester (39 g) und Natriummethylat (17 g) in 400 ml absolutem Toluol wurde 4 h unter Rühren und Rückfluß zum Sieden erhitzt (vgl. Ind. Eng. Chem. 1942, S. 494). Nach Zugabe von 300 ml Wasser wurde das Gemisch kurz gerührt. Es entstand ein Niederschlag (Natrium-Enolat des Produktes) zwischen den beiden Phasen. Es wurde auf Raumtemperatur abkühlen gelassen und anschließend wurde der gebildete Feststoff abgesaugt, welcher mit Ether und Wasser gewaschen wurde. Der Feststoff wurde in 500 ml Wasser in der Siedehitze gelöst und mit konz. Salzsäure angesäuert. Nach Abkühlen unter Rühren wurde der gebildete Feststoff abgesaugt und bei 60°C getrocknet. Das hellgelbe Produkt (22 g) wurde mittels NMR-Spektroskopie als 2-(3-Methoxyphenylacetyl)-indan-1,3-dion identifiziert.
iv) Das in Schritt iii) erhaltene Reaktionsprodukt (13 g) wurde in 200 ml Phosphorsäure suspendiert und unter Rühren in ein auf 120 °C vorgeheiztes Ölbad gestellt. Nach 1,5 h bei 120°C hatte sich eine orangefarbene Suspension gebildet. Nach dem Abkühlen wurde das Reaktionsgemisch in 800 ml Wasser unter Rühren eingegossen und die Suspension abgesaugt. Der Filterrückstand wurde gründlich mit Wasser gewaschen und bei 60°C getrocknet. Das orangefarbene Produkt (12 g) wurde mittels NMR-Spektroskopie als 6-Hydroxy-3-methoxy-7H-benzo[c]fluoren-7-on identifiziert.
v) 3 g des in Schritt iv) erhaltenen Reaktionsproduktes wurden zusammen mit 1-(4-Methoxyphenyl)-1-phenyl-1-propinol (4 g; hergestellt aus 4-Methoxybenzophenon und Natriumacetylid in DMSO) in etwa 300 ml Toluol suspendiert. Nach Zusatz einer Spatelspitze 4-Toluolsulfonsäure wurde das Reaktionsgemisch 1,5 h unter Rühren und Rückfluß zum Sieden erhitzt. Nach kurzem Abkühlen wurde das Toluol im Vakuum abgezogen und der Rückstand in 40 ml Dichlormethan gelöst und einer Säulenchromatographie mit Aluminiumoxid (Wassergehalt 3%) als stationärer und Dichlormethan/Hexan-Mischung (2:1) als mobiler Phase unterworfen. Die rote Produktbande wurde gesammelt. Nach Abziehen des Lösungsmittels wurde ein dunkelrotes Produkt (3 g) erhalten, das mittels NMR-Spektroskopie als 2-(4-Methoxyphenyl)-2-phenyl-6-methoxy-13-oxo-indeno[2,1-f]-naphtho[2,1-b]pyran identifiziert wurde.
vi) 2 g des in Schritt v) erhaltenen Reaktionsproduktes wurden in 50 ml absolutem THF unter Rühren gelöst. Zu dieser Lösung wurden 2 Äquivalente 2-Biphenylylmagnesiumbromid (hergestellt aus 2-Brombiphenyl und Magnesiumspäne in THF-Lösung) zugetropft. Das Reaktionsgemisch wurde bei Raumtemperatur 1 h gerührt. Anschließend wurde das Reaktionsgemisch in Wasser gegossen, mit konz. Salzsäure solange angesäuert, bis die Phasen klar waren, und die organische Phase abgetrennt. Nach Extraktion mit Wasser, Trocknen über Natriumsulfat und Abziehen des Lösungsmittel wurde ein bräunliches Öl (2 g) erhalten, das mittels NMR-Spektroskopie als 13-(2-Biphenylyl)-13-hydroxy-6-methoxy-2-(4-methoxyphenyl)-2-phenyl-indeno[2,1-f]naphtho[2,1-b]pyran identifiziert wurde.
vii) 1 g des in Schritt vi) erhaltenen Reaktionsproduktes wurden in 30 ml Eisessig in der Hitze cyclisiert. Nach Zugabe eines Tropfens Salzsäure wurde noch 5 min zum Sieden erhitzt und noch solange heißes Wasser zugegeben, bis die Reaktionslösung trüb wurde. Nach dem Abkühlen wurde mit Ether extrahiert. Die organische Phase wurde mit Wasser und verdünnter Natriumhydrogencarbonat-Lösung gewaschen. Nach Trocknen über Natriumsulfat wurde das Lösungsmittel abdestilliert und das Rohprodukt in 40 ml Dichlormethan gelöst und einer Säulenchromatographie mit Aluminiumoxid (Wassergehalt 3%) als stationärer und Dichlormethan/Hexan-Mischung (2:1) als mobiler Phase unterworfen. Aus der Produktfraktion wurde nach Abdestillieren des Eluenten ein Feststoff (0,5 g) erhalten, der mittels NMR-Spektroskopie als Spiro-9-fluoren-13'-[6-methoxy-2-(4-methoxyphenyl)-2-phenyl-indeno[2,1-f]naphtho[2,1-b]pyran] identifiziert wurde.

### BEISPIEL 2

Die Durchführung erfolgte analog Beispiel 1, mit der Ausnahme, dass im Schritt vi) die Umsetzung mit 2-Phenoxyphenylmagnesiumbromid (hergestellt aus 2-Bromdiphenylether und Magnesium in THF-Lösung) anstatt mit 2-Biphenylylmagnesiumbromid erfolgte. Gemäß NMR-Spektroskopie war das Endprodukt 13-Hydroxy-6-methoxy-2-(4-methoxyphenyl)-13-(2-phenoxyphenyl)-2-phenyl-indeno-[2,1-f]-naphtho[2,1-b]pyran.

Dieses wurde analog dem Schritt vii) von Beispiel 1 zu Spiro-9-xanthen-13'-[6-methoxy-2-(4-methoxyphenyl)-2-phenyl-indeno[2,1-f]naphtho[2,1-b]pyran] (0,4 g) cyclisiert, das mittels NMR-Spektroskopie identifiziert wurde.

### BEISPIEL 3

Die Durchführung erfolgte analog Beispiel 1, mit der Ausnahme, dass im Schritt vi) die Umsetzung mit 2-Benzylphenylmagnesiumbromid (hergestellt aus 2-Bromdiphenylmethan und Magnesium in THF-Lösung) anstatt 2-Biphenylylmagnesiumbromid erfolgte. Gemäß NMR-Spektroskopie entstand 13-(2-Benzylphenyl)-13-hydroxy-6-methoxy-2-(4-methoxyphenyl)-2-phenyl-indeno[2,1-f]-naphtho[2,1-b]pyran.

Dieses wurde analog dem Schritt vii) von Beispiel 1 zu Spiro-9-(9,10-dihydroanthracen)-13'-[6-methoxy-2-(4-methoxyphenyl)-2-phenyl-indeno[2,1-f]-naphtho-[2,1-b]-pyran] (0,5 g) cyclisiert, das mittels NMR-Spektroskopie identifiziert wurde.

### BEISPIEL 4

Die Durchführung erfolgt analog Beispiel 1, mit der Ausnahme, daß im Schritt i) die Umsetzung mit 3,4-Dimethoxybenzylcyanid anstatt 3-Methoxybenzylcyanid erfolgte. Es entstand nach Schritt vi) 13-(2-Biphenylyl)-6,7-dimethoxy-13-hydroxy-2-(4-methoxyphenyl)-2-phenyl-indeno[2,1-f]-naphtho[2,1-b]pyran und nach Cyclisierung analog dem Schritt vii) von Beispiel 1 Spiro-9-fluoren-13'-[6,7-dimethoxy-2-(4-methoxyphenyl)-2-phenyl-indeno[2,1-f]naphtho[2,1-b]pyran] (0,6 g), das mittels NMR-Spektroskopie identifiziert wurde.

### Herstellung der Probekörper:

500 ppm des jeweiligen photochromen Farbstoffes wurden im verwendeten Monomer (TRANSHADE-150 der Firma Tokuyama; Brechungsindex 1,52) bei Raumtemperatur unter Rühren gelöst. Nach Zugabe eines Initiators vom Alkylperoxyester-Typ (1,5 Gew.-%) wurde zweimal entgast und anschließend nach dem von der Firma Tokuyama empfohlenen Temperaturprogramm polymerisiert. Die Glasgießformen waren dabei schwarz gefärbt, damit die Gesamtheit der photochromen Farbstoffe im nichtangeregten Zustand in die Matrix eingebaut werden konnte. Nach Beendigung der Polymerisation wurden die Probekörper noch 2 h bei 100°C getempert.

### Ermittlung der Kinetikwerte sowie der längstwelligen Absorptionsmaxima:

Zur Ermittlung der Aufhellgeschwindigkeiten wurden die derart hergestellten Probekörper in einer Kinetikbank PTM II der Fa. Zeiss vermessen (Bestrahlung mit 50 klux gemäß DIN EN 1836 Punkt 6.1.3.1.1.). Die Belichtungszeit betrug jeweils 15 min, die Aufhellung im Dunkeln 10 min. Die Temperatur des Glases wurde über eine thermostatisierbare Küvette geregelt. Während der Belichtung und der Aufhellung wurde die Transmission - bewertet nach der Hellempfindlichkeit des menschlichen Auges V(λ) - jeweils in kurzen Zeitintervallen aufgezeichnet. Darüberhinaus liefert die PTM II-Kinetikbank zum Belichtungsende das UV/VIS-Spektrum der eingedunkelten Probekörper, woraus die längstwelligen Absorptionsmaxima ermittelt werden können.

In der nachstehenden Tabelle 1 sind die längstwelligen Absorptionsmaxima im eingedunkelten Zustand sowie die Halbwertszeit der Aufhellung, d.h. die Zeit von der maximaler Eindunklung bis zu einer Transmission, die äquidistant zur maximalen Eindunklung und Aufhellung liegt, der vorstehend hergestellten erfindungsgemäßen Spiroverbindungen sowie einer im Stand der Technik bekannten Vergleichsverbindung, wie in US-A 5,869,658 beschrieben, welche die nachstehende Strukturformel (III) aufweist: 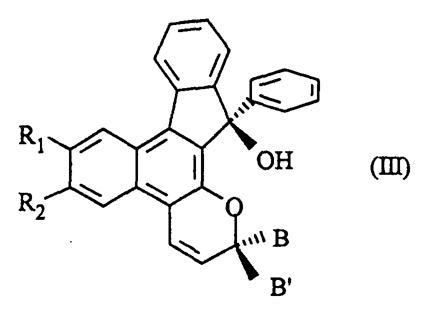 aufgeführt.

**TABELLE 1**

| | R₁ | R₂ | B | B' | X | λₘₐₓ | Halbwertszeit Aufhellung |
|---|---|---|---|---|---|---|---|
| Beispiel 1 | OMe | H | -C₆H₄OMe | H | - | 455 nm | 1 min |
| Beispiel 2 | OMe | H | -C₆H₄OMe | H | O | 460 nm | 2 min |
| Beispiel 3 | OMe | H | -C₆H₄OMe | H | CH₂ | 455 nm | 3 min |
| Beispiel 4 | OMe | OMe | -C₆H₄OMe | H | - | 465 nm | 5 min |
| Vergleichsbeispiel aus US-A 5,869,658 | OMe | OMe | -C₆H₄OMe | H | vgl. Formel (III) | 460 nm | 8 min |

Aus der Tabelle 1 ist die schnellere Aufhellung der erfindungsgemäßen Spiro-Verbindungen im Vergleich zu der im Stand der Technik verfügbaren Verbindung, wie in US-A 5,869,658 beschrieben, deutlich ersichtlich.

## Patentansprüche

1. Photochrome 3H-Naphtho-[2,1-b]pyrane mit der allgemeinen Formel (I): 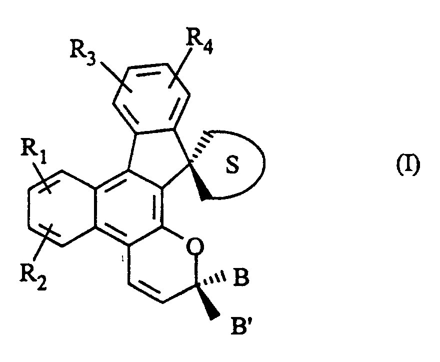 worin
die Reste R₁, R₂, R₃ und R₄ jeweils unabhängig voneinander einen Substituenten darstellen, ausgewählt aus der Gruppe A, bestehend aus einem Wasserstoffatom, einem (C₁-C₆)-Alkylrest, einem (C₁-C₆)-Alkoxyrest, einem (C₃-C₇)-Cycloalkylrest, der ein oder mehrere Heteroatome aufweisen kann, einem Phenylrest, einer Hydroxygruppe, Brom, Chlor und Fluor,
die Struktureinheit S unter Einbeziehen des zentralen Spirokohlenstoffatoms ein gesättigtes und/oder ungesättigtes Ringglied mit 5 bis 8 Kohlenstoffatomen darstellt, von denen maximal eines durch ein Heteroatom, ausgewählt aus der Gruppe, bestehend aus O, S und NR₅, ersetzt sein kann, wobei der Rest R₅ einen geradkettigen oder verzweigtkettigen (C₁-C₆)-Alkylrest, einen substituierten oder unsubstituierten (C₅-C₇)-Cycloalkylrest, einen substituierten oder unsubstituierten Phenylrest oder einen substituierten oder unsubstituierten Benzylrest darstellt, wobei an das Ringglied mindestens ein aromatisches oder heteroaromatisches Ringsystem annelliert ist, wobei das Ringsystem ausgewählt ist aus der Gruppe E, bestehend aus Benzol, Naphthalin, Phenanthren, Pyridin, Chinolin, Furan, Thiophen, Pyrrol, Benzofuran, Benzothiophen, Indol und Carbazol, und das Ringsystem einen oder mehrere Substituenten aus der Gruppe A aufweisen kann;
B und B' unabhängig voneinander aus einer der folgenden Gruppen a), b), c) oder d) ausgewählt sind, wobei sie
a) mono-, di- und trisubstituierte Arylreste sind, wobei der Arylrest Phenyl oder Naphthyl ist;
b) unsubstituierte, mono- und disubstituierte Heteroarylreste sind, wobei der Heteroarylrest Pyridyl, Furanyl, Benzofuran-2-yl, Benzofuran-3-yl, Thienyl, Benzothien-2-yl oder Benzothien-3-yl ist;
wobei die Substituenten der Aryl- oder Heteroarylreste in a) und b) solche sind, ausgewählt aus einer Gruppe, bestehend aus Hydroxy, Amino, Mono-(C₁-C₆)-alkylamino, Di-(C₁-C₆)-alkylamino, am Aromaten un-, mono- oder disubstituiertem Mono- und Diphenylamino, Piperidinyl, Morpholinyl, Carbazolyl, un-, mono- und disubstituiertem Pyrryl, (C₁-C₆)-Alkyl, (C₁-C₆)-Alkoxy, Brom, Chlor und Fluor, wobei die vorgenannten aromatischen und heteroaromatischen Ringsysteme mit (C₁-C₆)-Alkyl, (C₁-C₆)-Alkoxy, Brom, Chlor und Fluor substituiert sein können;
c) Struktureinheiten mit den folgenden Formeln (V) und (W) sind 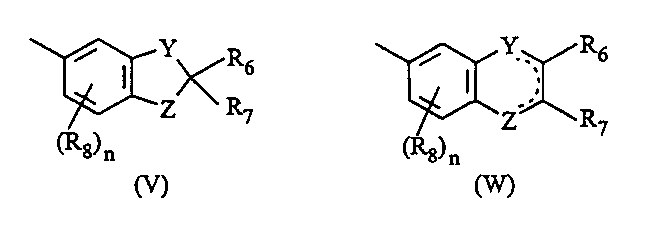 worin
Y und Z unabhängig voneinander O, S, CH, CH₂ oder NR₉ sind, wobei der Rest R₉ ausgewählt ist aus der Gruppe D, bestehend aus (C₁-C₆)-Alkyl, (C₁-C₆)-Acyl und Wasserstoff, die Reste R₆ und R₇ unabhängig voneinander Wasserstoff und/oder einen (C₁-C₆)-Alkylrest darstellen und der Rest R₈ ein Substituent aus der Gruppe A ist, wobei n 1, 2, oder 3 ist, mit der Maßgabe, daß, wenn Y in der Formel (V) NR₉ ist, Z Kohlenstoff ist, oder
d) B und B' miteinander einen un-, mono- oder disubstituierten Fluoren-9-ylidenrest oder einen gesättigten Kohlenwasserstoffrest bilden, der C₃-C₁₂ spiro-monozyklisch, C₇-C₁₂ spiro-bizyklisch und/oder C₇-C₁₂ spiro-trizyklisch ist, wobei die Fluorensubstituenten aus der Gruppe A ausgewählt sind.

2. Photochrome 3H-Naphtho-[2,1-b]pyrane nach Anspruch 1, wobei in der vorstehenden Formel (I) die Struktureinheit S unter Einbeziehen des zentralen Spirokohlenstoffatoms eine substituierte oder unsubstituierte Fluoren-, Xanthen-, Thioxanthen- oder Dihydroanthracen-Einheit ist.

3. Photochrome 3H-Naphtho-[2,1-b]pyrane nach Anspruch 1 oder 2, welche die allgemeine Formeln (II) aufweisen: 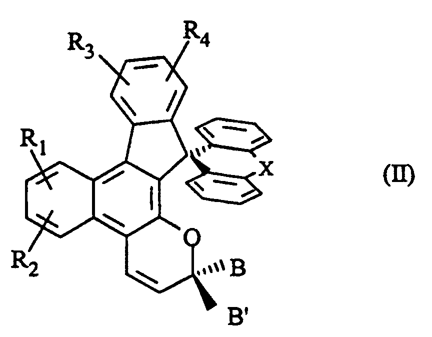 worin R₁, R₂, R₃ und R₄ wie vorgenannt definiert sind und X für -CH₂-, -O-, -S- oder eine C-C-Einfachbindung zwischen den Phenylringen steht.

4. Photochrome 3H-Naphtho-[2,1-b]pyrane nach Anspruch 3, wobei in der vorstehend aufgeführten Formel (II) B und B' unabhängig voneinander mono-, di- und trisubstituierte Arylreste sind, wobei der Arylrest jeweils ein Phenylrest oder ein Naphthylrest ist.

5. Photochrome 3H-Naphtho-[2,1-b]pyrane nach einem der vorgehenden Ansprüche, welche
Spiro-9-fluoren-1 3'-[6-methoxy-2-(4-methoxyphenyl)-2-phenyl-indeno[2,1-f]-naphtho[2,1-b]pyran],
Spiro-9-xanthen-13'-[6-methoxy-2-(4-methoxyphenyl)-2-phenyl-indeno[2,1-f]-naphtho[2,1-b]pyran],
Spiro-9-thioxanthen-13'-[6-methoxy-2-(4-methoxyphenyl)-2-phenyl-indeno-[2,1-f]-naphtho[2,1-b]pyran],
Spiro-9-(9,10-dihydroanthracen)-13'-[6-methoxy-2-(4-methoxyphenyl)-2-phenyl-indeno[2,1-f]naphtho[2,1-b]pyran] und
Spiro-9-fluoren-13'-[6,7-dimethoxy-2-(4-methoxyphenyl)-2-phenyl-indeno-[2,1f]-naphtho[2,1-b]pyran] sind.

6. Verwendung der photochromen 3H-Naphtho-[2,1-b]pyrane nach einem der Ansprüche 1 bis 5 in Kunststoffmaterialien.

7. Verwendung nach Anspruch 6, wobei das Kunststoffmaterial eine ophthalmische Linse ist.

## Claims

1. Photochromic 3H-naphtho[2,1-b]pyrans with the general formula (I): 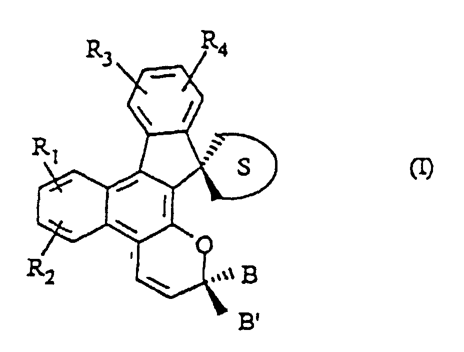 in which
the radicals R₁, R₂, R₃ and R₄, respectively independently of each other, represent a substituent which is chosen from the group A which comprises a hydrogen atom, a (C₁-C₆)alkyl radical, a (C₁-C₆)alkoxy radical, a (C₃-C₇)cycloalkyl radical, which can have one or more heteroatoms, a phenyl radical, a hydroxy group, bromine, chlorine and fluorine;
the structural unit S including the central spirocarbon atom represents a saturated and/or unsaturated ring member with 5 to 8 carbon atoms, of which a maximum of one atom can be replaced by a heteroatom which is chosen from the group which comprises O, S and NRs, the radical R₅ representing an unbranched-chain or branched-chain (C₁-C₆)alkyl radical, a substituted or unsubstituted (C₅-C₇)cycloalkyl radical, a substituted or unsubstituted phenyl radical or a substituted or unsubstituted benzyl radical, at least one aromatic or heteroaromatic ring system being anellated on the ring member, the ring system being chosen from the group E which comprises benzene, naphthalene, phenanthrene, pyridine, quinoline, furane, thiophene, pyrrole, benzofurane, benzothiophene, indole and carbazole, and the ring system being able to have one or more substituents from the group A;
B and B', independently of each other, are chosen from one of the following groups a), b), c) or d), these
a) being mono-, di- and tri-substituted aryl radicals, the aryl radical being phenyl or naphthyl;
b) being unsubstituted, mono- and di-substituted heteroaryl radicals, the heteroaryl radical being pyridyl, furanyl, benzofurane-2-yl, benzofurane-3-yl, thienyl, benzothien-2-yl or benzothien-3-yl; the substituents of the aryl or heteroaryl radicals in a) and b), which are chosen from a group which comprises hydroxy, amino, mono-(C₁-C₆)alkylamino, di-(C₁-C₆)alkylamino, being those on the aromatic un-, mono- or di-substituted mono- and diphenylamino, piperidinyl, morpholinyl, carbazolyl, un-, mono- and di-substituted pyrryl, (C₁-C₆)alkyl, (C₁-C₆)alkoxy, bromine, chlorine and fluorine, the aforementioned aromatic and heteroaromatic ring systems being able to be substituted with (C₁-C₆)alkyl, (C₁-C₆)alkoxy, bromine, chlorine and fluorine;
c) structural units with the following formulae (V) and (W) being 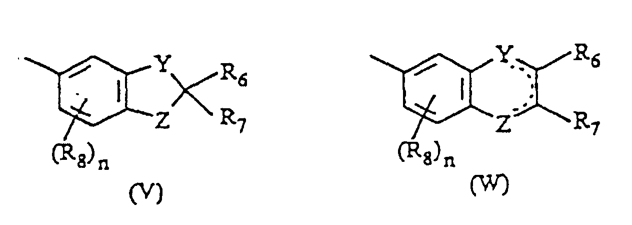 in which
Y and Z independently of each other are O, S, CH, CH₂ or NR₉, the radical R₉ being chosen from the group D which comprises (C₁-C₆)alkyl, (C₁-C₆)acyl and hydrogen, the radicals R₆ and R₇ independently of each other representing hydrogen and/or a (C₁-C₆)alkyl radical, and the radical R₈ is a substituent from the group A, n being 1, 2 or 3, with the proviso that if Y is NR₉ in the formula (V), Z is carbon,
or
d) B and B' together forming an un-, mono- or di-substituted fluorene-9-ylide radical or a saturated hydrocarbon radical, which is C₃-C₁₂ spiro-monocyclic, C₇-C₁₂ spiro-bicylic and/or C₇-C₁₂ spiro-tricyclic, the fluorene substituents being chosen from the group A.

2. Photochromic 3H-naphtho[2,1-b]pyrans according to claim 1, the structural unit S in the preceding formula (I) including the central spirocarbon atom being a substituted or unsubstituted fluorene, xanthene, thioxanthene or dihydroanthracene unit.

3. Photochromic 3H-naphtho[2,1-b]pyrans according to claim 1 or 2, which have the general formula (II): 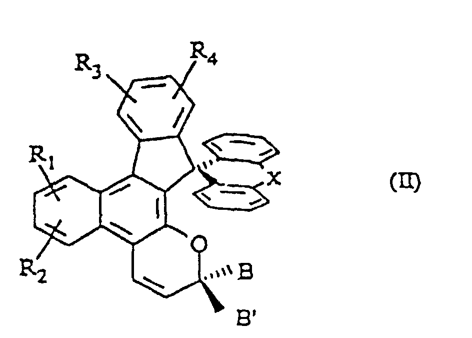 in which R₁, R₂, R₃ and R₄ are defined as mentioned above, and X stands for -CH₂-, -O-, -S- or a C-C single bond between the phenyl rings.

4. Photochromic 3H-naphtho[2,1-b]pyrans according to claim 3, B and B' in the above cited formula (II) independently of each other being mono-, di- and tri-substituted aryl radicals, the aryl radical being respectively a phenyl radical or a naphthyl radical.

5. Photochromic 3H-naphtho[2,1-b]pyrans according to one of the preceding claims, which are
spiro-9-fluorene-13'-[6-methoxy-2-(4-methoxyphenyl)-2-phenyl-indeno[2,1-f]-naphtho[2,1-b]pyran],
spiro-9-xanthene-13'-[6-methoxy-2-(4-methoxyphenyl)-2-phenyl-indeno[2,1-f]-naphtho[2,1-b]pyran],
spiro-9-thioxanthene-13'-[6-methoxy-2-(4-methoxyphenyl)-2-phenyl-indeno[2,1-f]-naphtho[2,1-b]pyran],
spiro-9-(9,10-dihydroanthracene)-13'-[6-methoxy-2-(4-methoxyphenyl]-2-phenyl-indeno[2,1-f]naphtho[2,1-b]pyran], and
spiro-9-fluorene-13'-[6,7-dimethoxy-2-(4-methoxyphenyl)-2-phenyl-indeno[2,1-f]-naphtho[2,1-b]pyran],

6. Use of the photochromic 3H-naphtho[2,1-b]pyrans according to one of the claims 1 to 5 in plastic materials.

7. Use according to claim 6, the plastic material being an ophthalmic lens.

## Revendications

1. 3H-naphto-[2,1-b]pyrane photochrome de formule générale (I) : 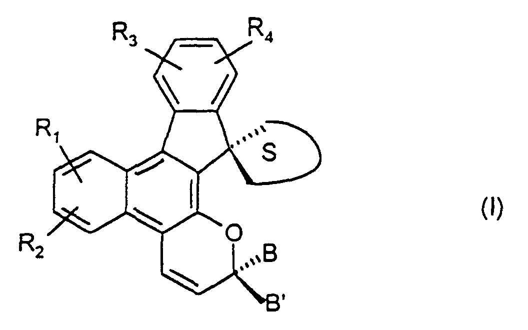 dans laquelle
les radicaux R₁, R₂, R₃ et R₄ représentent, indépendamment les uns des autres, un substituant, choisi parmi le groupe A, constitué d'un atome d'hydrogène, d'un radical alkyle en (C₁-C₆), d'un radical alcoxy en (C₁-C₆), d'un radical cycloalkyle en (C₃-C₇) présentant un ou plusieurs hétéroatomes, un radical phényle, un groupe hydroxy, le brome, chlore et fluor,
l'unité structurelle S, en intégrant l'atome central de spirocarbone, représente un élément du cycle saturé et/ou insaturé doté de 5 à 8 atomes de carbone, parmi lesquels au maximum un de ceux-ci peut être substitué par un hétéroatome, choisi parmi le groupe O, S et NR₅, le radical R₅ représentant un radical alkyle en (C₁-C₆) à chaîne linéaire ou ramifiée, un radical cycloalkyle en (C₅-C₇) substitué ou non substitué, un radical phényle substitué ou non substitué ou un radical benzyle substitué ou non substitué, au moins un système cyclique aromatique ou hétéroaromatique étant ajouté à l'élément du cycle, le système cyclique étant choisi parmi le groupe E, constitué du benzène, de la naphtaline, du phénanthrène, de la pyridine, de la quinoline, du furanne, du thiophène, du pyrrol, du benzofuranne, du benzothiophène, de l'indole et du carbazole et le système cyclique pouvant présenter un ou plusieurs substituants du groupe A ;
B et B' sont choisis, indépendamment l'un de l'autre, parmi l'un des groupes suivants a), b), c) ou d),
a) étant des radicaux aryle mono-, di- et trisubstitués, le radical aryle étant le phényle ou le naphthyle ;
b) étant des radicaux hétéroaryle non substitués, mono- et disubstitués, le radical hétéroaryle étant le pyridyle, le furanyle, le benzofurane-2-yle, le benzofurane-3-yle, le thiényle, le benzothiène-2-yle ou le benzothiène-3-yle ;
les substituants des radicaux aryle ou hétéroaryle dans a) et b) étant ceux choisis parmi un groupe constitué du groupe hydroxy, amino, mono-(C₁-C₆)-alkylamino, di-(C₁-C₆)-alkylamino, mono- et diphénylamino non substitué, mono- ou disubstitué au niveau de l'aromate, pipéridinyle, morpholinyle, carbazolyle, pyrryle non substitué, mono- et disubstitué, alkyle en (C₁-C₆), alcoxy en (C₁-C₆), brome, chlore et fluor, les systèmes cycliques aromatiques et hétéroaromatiques précédents pouvant être substitués par un groupe alkyle en (C₁-C₆), alcoxy en (C₁-C₆), brome, chlore et fluor ;
c) les unités structurelles de formules suivantes (V) et (W) étant 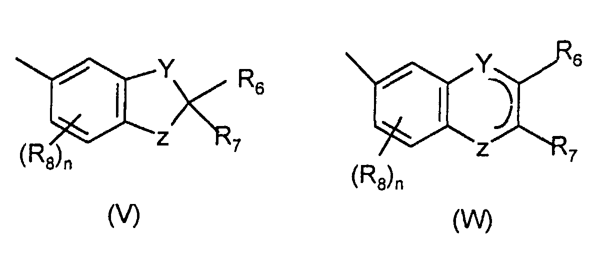 dans lesquelles
Y et Z, indépendamment l'un de l'autre, étant O, S, CH, CH₂ ou NR₉, le radical R₉ étant choisi parmi le groupe D, constitué du groupe alkyle en (C₁-C₆), acyle en (C₁-C₆) et hydrogène, les radicaux R₆ et R₇, indépendamment l'un de l'autre, représentant l'hydrogène et/ou un radical alkyle en (C₁-C₆) et le radical R₈ étant un substituant du groupe A, n étant égal à 1, 2 ou 3, à la condition que si Y est R₉ dans la formule (V), Z est le carbone,
ou
d) B et B' ensemble formant un radical fluorène-9-ylidène non substitué, mono- ou disubstitué ou un radical hydrocarbure saturé, qui est spiro-monocyclique en C₃-C₁₂, spiro-bicyclique en C₇-C₁₂ et/ou spiro-tricyclique en C₇-C₁₂, les substituants fluorène étant choisis parmi le groupe A.

2. 3H-naph-to-[2,1-b]pyrane photochrome selon la revendication 1, l'unité structurelle S, en intégrant l'atome de spirocarbone central, étant dans la formule (I) précédente une unité fluorène, xanthène, thioxanthène ou dihydroanthracène substituée ou non substituée.

3. 3H-naphto-[2,1-b]pyrane photochrome selon la revendication 1 ou 2, lequel présente la formule générale (II) : 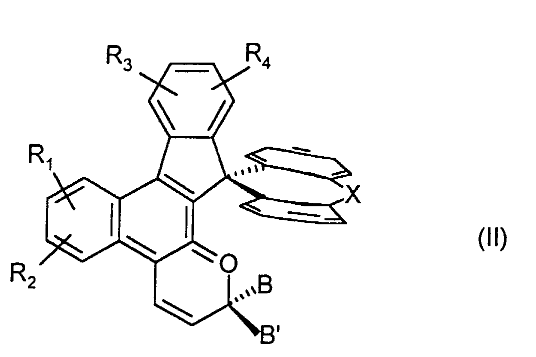 dans laquelle R₁, R₂, R₃ et R₄ sont tels que définis précédemment, et X représente -CH₂-, -O-, -S- ou une simple liaison C-C entre les cycles phényle.

4. 3H-naphto-[2,1-b]pyrane photochrome selon la revendication 3, B et B' étant, indépendamment l'un de l'autre, dans la formule (II) énoncée précédemment des radicaux aryle mono-, di- ou trisubstitués, le radical aryle étant respectivement un radical phényle ou un radical naphtyle.

5. 3H-naphto-[2,1-b]pyrane photochrome selon l'une des revendications précédentes, lequel étant le :
spiro-9-fluorène-13'-[6-méthoxy-2-(4-méthoxyphényl)-2-phényl-indèno[2,1-f]-naphto[2,1-b]pyrane],
spiro-9-xanthène-13'-[6-méthoxy-2-(4-méthoxyphényl)-2-phényl-indèno[2,1-f]-naphto[2,1-b]pyrane],
spiro-9-thioxanthène-13'-[6-méthoxy-2-(4-méthoxyphényl)-2-phényl-indèno[2,1-f]-naphto[2,1-b]pyrane],
spiro-9-(9,10-dihydroanthracène)-13'-[6-méthoxy-2-(4-méthoxyphényl)-2-phényl-indèno[2,1-f]-naphto[2,1-b]pyrane],
spiro-9-fluorène-13'-[6,7-diméthoxy-2-(4-méthoxyphényl)-2-phényl-indèno[2,1-f]-naphto[2,1-b]pyrane].

6. Utilisation du 3H-naphto-[2,1-b]pyrane photochrome selon l'une des revendications 1 à 5 dans les matières plastiques.

7. Utilisation selon la revendication 6, dans laquelle la matière plastique est une lentille optique.
